# EUROPEAN PATENT SPECIFICATION

(11) **EP 2 999 514 B1**
(45) Date of publication and mention of the grant of the patent: **11.12.2024**
(21) Application number: 14801270.1
(22) Date of filing: 20.05.2014
(51) Int. Cl.: A61N 1/36

(54) **METHODS FOR DEEP BRAIN STIMULATION PARAMETERS**
VERFAHREN FÜR PARAMETER ZUR TIEFEN HIRNSTIMULATION
PROCÉDÉS POUR DES PARAMÈTRES DE STIMULATION CÉRÉBRALE PROFONDE

(30) Priority: 21.05.2013 US 201361825692 P; 22.05.2013 US 201361826077 P; 22.05.2013 US 201361826201 P
(43) Date of publication of application: 30.03.2016
(73) Proprietor: Duke University, Durham, NC 27705 (US)
(72) Inventor: GRILL, Warren M., Chapel Hill, North Carolina 27516 (US); BROCKER, David T., Triangle, VA 22172-131 (US); KENT, Alexander R., Durham, North Carolina 27705 (US)
(74) Representative: Müller-Boré & Partner Patentanwälte PartG mbB
(86) International application number: PCT/US2014/038809
(87) International publication number: WO 2014/189944

(56) References cited:
- US-A1- 2006 017 749
- US-A1- 2010 152 807
- US-A1- 2011 270 348
- No further relevant documents disclosed

## Description

The present invention relates generally to the field of deep brain stimulation using devices, systems and methods for tuning non-regular temporal patterns of brain stimulation. Specifically, the present invention relates to methods for generating a brain stimulation pattern for treating a patient with a neurological disorder. Deep Brain Stimulation (DBS) has been found to be successful in treating a variety of neurological disorders, including movement disorders. Generally, such treatment involves placement of a DBS type lead into a targeted region of the brain through a burr hole drilled in the patient's skull, and the application of appropriate stimulation through the lead to the targeted region.

Oscillatory and synchronous neural activity appears to be the cause of many neurological disorders and may be important for the proper functioning of brain structures. While existing systems and methods can provide benefits to patients suffering from neurological disorders, many quality of life issues still remain. Current systems and methods of deep brain stimulation rely on process that use a fixed temporal pattern of stimulation or randomly generates a pattern of stimulation.

Pfaff and colleagues have proposed using non-regular patterns of stimulation in mammals, including humans, that are comprised of a series of pulses whose interpulse intervals are varied using a non-linear dynamical system (Pfaff and Quinkert; 2010). Their stimulation methods seem to be effective in an arousal model of a minimally conscience state (Quinkert and Pfaff; 2012). These patterns of stimulation are essentially randomly generated, rather than designed for a specific disorder and symptom(s). The devices, systems and methods described herein use model-based design to create specific patterns of stimulation.

Feng et al. used an optimization protocol in a model that showed, incorrectly, symptom reduction with regular, low frequency stimulation (10 Hz) that previous clinical work has demonstrated to be ineffective. The Feng et al. model was not validated. Also, the Feng et al. stimulation patterns are generated by an optimization protocol that generates parameters defining a stochastic process. That stochastic process, in turn, randomly generates a pattern of stimulation. Feng et al. used a genetic algorithm to design a stochastic process that is capable of generating a random pattern of stimulation that performs well in their non-validated model.
In this context, US 2011/0270348 A1 describes a programming system allowing a user to program therapy parameter values for therapy delivered by a medical device.

The present invention relates to the embodiments characterized in the appended claims. Any embodiment, which is in contradiction to the subject-matter of claim 1, is not part of the invention.
Oscillatory and synchronous neural activity appears to be the cause of many neurological disorders and may be important for the proper functioning of brain structures. There is evidence that the spectral characteristics of neural activity differ across symptoms. Therefore, the invention described herein is useful to patients with neurological disorders, including movement disorders, because it may provide customized stimulation parameters based on specific symptoms. The customized stimulation may be more effective and/or efficient than regular high frequency stimulation and/or stimulation patterns described previously. Furthermore, the temporal pattern of stimulation may be adjusted as changes in symptoms occur, perhaps because of disease progression or changes due to medications.

Described herein are systems and methods for the design and application of specific temporal non-regular patterns of stimulation according to the symptom experienced by a patient with a neurological disorder or proxies for symptom.

One aspect described herein provides for increasing the efficacy and/or efficiency of deep brain stimulation (DBS) using parameters of stimulation that are custom tailored to a unique set of one or more symptoms.

One aspect of the present teachings provides a method for generating a brain stimulation pattern for treating a patient with a neurological disorder comprising, consisting of, or consisting essentially of identifying at least one symptom of the neurological disorder, and optimizing a computational model of the brain stimulation pattern to suppress oscillations in a model associated with the at least one symptom of the neurological disorder.

One aspect described herein provides methods for generating brain stimulation parameters to treat specific symptoms of a neurological disorder comprising, consisting of, or consisting essentially of optimizing, using a computational model, the temporal patterns of stimulation to suppress oscillations associated with the specific symptom.

Another aspect of the present disclosure provides methods of treating one or more specific symptoms in a subject suffering from a neurological disorder comprising, consisting of, or consisting essentially of optimizing, using a computational model, the temporal patterns of stimulation to suppress oscillations associated with the specific symptom; configuring a pulse generator with the optimized parameters; and delivering to the subject the pattern of stimulation thereby treating the one or specific symptoms.

Another aspect of the present disclosure provides method for treating a symptom of a neurological disorder, the method comprising, consisting of or consisting essentially of identifying at least one symptom of the neurological disorder in a patient, generating a brain stimulation pattern by optimizing a computational model of the stimulation pattern to suppress oscillations in a model associated with the at least one symptom of the neurological disorder, configuring a pulse generator with the brain stimulation pattern, and delivering to the patient the brain stimulation pattern with the pulse generator, thereby treating the at least one symptom of the neurological disorder.

One aspect of the present disclosure provides methods for generating patient-specific tuning of brain stimulation parameters comprising, consisting of, or consisting essentially of (a) recording neural activity of the patient to generate a patient-specific neural activity; (b) tuning a model to reproduce the observed patient-specific neural activity; and (c) optimizing the parameters of stimulation using the tuned model.

One aspect of the present disclosure provides a method for generating a brain stimulation pattern for treating a patient with a neurological disorder comprising, consisting of or essentially consisting of recording the neural activity of the patient, tuning a computational model of the stimulation pattern to reproduce the observed neural activity, and optimizing the computational model of the stimulation pattern using the tuned model.

Another aspect of the present disclosure provides methods of delivering patient-specific tuning of brain stimulation parameters to a subject suffering from a neurological disorder comprising, consisting of, or consisting essentially of recording neural activity of the patient to generate a patient-specific neural activity, tuning a model to reproduce the observed patient-specific neural activity, optimizing the parameters of stimulation using the tuned model; and configuring a pulse generator with the optimized parameters to administer stimulation to the patient to disrupt or promote oscillatory or synchronous activity.

Another aspect of the present disclosure provides a method for treating a patient having a neurological disorder comprising, consisting of or consisting essentially of recording neural activity of the patient, tuning a computational model of a stimulation pattern to reproduce the observed neural activity, optimizing the computational model of the stimulation pattern using the tuned model, configuring a pulse generator with the optimized stimulation pattern, and delivering to the patient the stimulation pattern with the pulse generator to disrupt or promote oscillatory or synchronous neural activity.

In some embodiments, the neurological disorder comprises Parkinson's Disease.

In some embodiments, the optimization comprises using an algorithm selected from the group consisting of an evolutionary algorithm, swarm intelligence algorithms and other optimization techniques. In some embodiments, the algorithm comprises an evolutionary algorithm. In certain embodiments, the algorithm comprises a Genetic Algorithm.

In some embodiments, the pulse generator is implantable.

Another aspect of the present disclosure provides for all that is disclosed and illustrated herein.

The present disclosure provides devices, systems and methods for recording pertinent neural •activity during any type of brain stimulation, including non-regular patterns of stimulation and processing techniques for these recorded signals and stimulation parameter optimization based on these recorded signals.

One aspect provides a device capable of recording neural activity during any type of brain stimulation comprising, consisting of, or consisting essentially of (a) a stimulating electrode with at least one stimulating contact; (b) at least one recording contact in electrical communication with a multi-stage series amplification device, the device may include a powered preamplifier to measure the differential signal from the recording contacts to reduce common-mode noise and at least two additional amplifier stages that are in series to increase gain and filter the signal; (c) a plurality of antiparallel diode clamps positioned at the inputs of the at least two additional amplifier stages; and (d) a parallel resistor positioned across the stimulating electrodes to allow accumulated charge on the stimulating contacts to discharge between pulses.

In some embodiments, the at least one recording contact is non-stimulating.

In other embodiments, the powered preamplifier is battery powered.

In yet another embodiment, the at least two additional amplifier stages are internally grounded through an opto-isolated CMOS multiplexer.

In other embodiments, the device is implantable.

Other aspects of the present disclosure provide systems comprising, consisting of or consisting essentially of the device and methods of making and using the device. Another aspect of the present disclosure provides for all that is disclosed and illustrated herein.

One aspect described herein may include a device that can deliver non-regular temporal patterns of stimulation and simultaneously record neural activity and mitigate the effects of the stimulus artifacts through amplifier blanking and/or stimulation relay.

Second, novel data processing techniques for this application are described in detail that can be used to overcome any bias or error introduced into signal characteristics of interest such as the spectral content
Third, novel applications for using the recorded neural activity as feedback to modulate the stimulation parameters are described. The temporal pattern of stimulation can be a stimulation parameter that is modified, optimized, or otherwise learned based on the recorded neural activity.

Operation of the invention may be better understood by reference to the following detailed description taken in connection with the following illustrations, wherein:
Figure 1 is a depiction of a computational model of the basal ganglia.
Figures 2A-2D are graphs of the evolution of oscillatory activity as current applied to the external globus pallidus is varied.
Figure 3 is a block diagram of the progression of the genetic algorithm.
Figure 4A and 4B are plots of example GA-designed non-regular pattern of stimulation for Parkinson's disease. Figure 4A is a plot of the performance of the non regular patterns of DBS. Figure 4B is a plot of the computational model compared to regular DBS.
Figure 5 is a diagram of the DBS-ECAP instrumentation used for stimulus artifact reduction and ECAP recording during DBS.

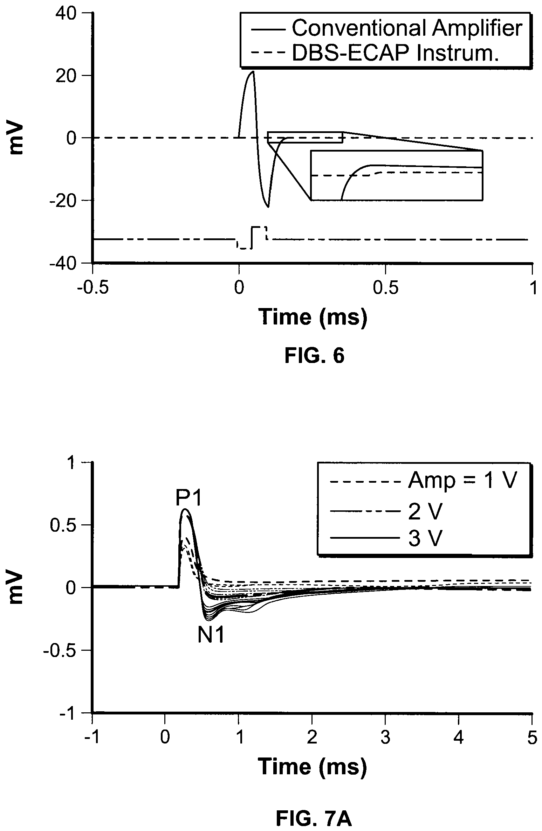

Figure 6 is a graph depicting *in vitro* stimulus artifact waveforms recorded from a clinical DBS electrode placed in a saline bath, using either a conventional biopotential amplifier (solid trace) or the DBS-ECAP instrumentation (dashed trace). The inset shows a zooned view of the waveforms. The timing of the DBS pulse in shown as the bottom dashed trace. The DBS-ECAP instrumentation eliminates the first two phases of the artifact, corresponding to the two phases of the DBS pulse, and reduces the third phase of the artifact, corresponding to capacitive discharging from the electrode-tissue interface of the stimulating electrodes.
Figures 7A-7C are graphs representing *in vivo* ECAP response measured across stimulation parameters and recorded from a mini DBS electrode implanted in the thalamus of a cat using the DBS-ECAP instrumentation. DBS was applied at time 0.
Figure 8 is a simplified block diagram of device components. Dotted lines indicate wireless communication. Dashed lines indicate optional components or connections.
Figure 9 is a flowchart illustrating the methodology for patient-specific tuning of brain stimulation parameters.

Articles "a" and "an" are used herein to refer to one or to more than one (i.e. at least one) of the grammatical object of the article. By way of example, "an element" means at least one element and can include more than one element.

Unless otherwise defined, all technical terms used herein have the same meaning as commonly understood by one of ordinary skill in the art to which this disclosure belongs.

Neural activity can be recorded from electrodes situated within the brain. The activity may serve as a biomarker for disease and provide insight into the stimulation parameters that should be used. Selection or optimization of non-regular temporal patterns of stimulation can be performed based on neural recordings from a patient. These recording can be conducted intermittently or on a continuous basis. Recording neural activity during stimulation can be very challenging, especially during non-regular patterns of stimulation because, contrary to regular high frequency (>100 Hz) stimulation, there is no guarantee of separation in the frequency domain between neural signals of interest (typically <100 Hz for local field potentials) and stimulation artifacts. Therefore, there is no opportunity to exploit this separation in the frequency domain and suppress stimulation artifacts via filtering (frequency domain solution).

Non-regular patterns of stimulation show great promise in more effectively and/or efficiently reducing symptoms of neurological disorders. The efficacy of the non-regular stimulation may depend on the ability to disrupt or otherwise change ongoing neural activity. Further, patterns may be optimized to disrupt certain pathological patterns of neural activity. These therapeutic approaches rely on the ability to record the underlying neural activity, even in the presence of non-regular stimulation. A novel device and methods for achieving this goal is described below. Further, applications for these neural recordings are described.

In one aspect of the present teachings, neural activity during non-regular stimulation may be recorded. A method for achieving these recordings while mitigating the deleterious effects of stimulation artifact is described. In another aspect described herein, applications of these recordings and methods for selection or optimization of temporal patterns of stimulation based on recorded signals are described.

In another aspect of the present teachings, non-regular patterns of deep brain stimulation may be applied in patients with neurological disorders, including Parkinson's Disease, movement disorders, epilepsy, and psychiatric disorders such as obsessive-compulsion disorder and depression. The non-regular stimulation patterns or trains may also be readily applied to other classes of electrical stimulation of the nervous system including, but not limited to, cortical stimulation, spinal cord stimulation, and peripheral nerve stimulation (including sensory and motor), to provide the attendant benefits described herein and to treat diseases such as but not limited to Parkinson's Disease, Essential Tremor, Movement Disorders, Dystonia, Epilepsy, Pain, psychiatric disorders such as Obsessive Compulsive Disorder, Depression, and Tourette's Syndrome.

Randomly generated patterns of stimulation with a high average frequency are ineffective at suppressing motor symptoms in essential tremor (ET) and Parkinson's disease (PD) (Birdno 2009, Dorval 2010). It was not until more structured patterns of stimulation designed to expose the effects of certain characteristics of the stimulation were tested that non-regular, high-frequency patterns of stimulation that significantly improved a measure of motor performance when compared to regular stimulation at a comparable frequency were found. (Brocker et al" 2013; Birdno et al., 2011).

In another aspect of the present teachings, non-regular, low frequency patterns of deep brain stimulation in patients with Parkinson's Disease may be applied. The methods used to generate non-regular, low frequency patterns of stimulation are described in U.S. Patent Publication No. 2013-0231715 and U.S. Patent No. 8,447,405.

The efficacy of these non-regular patterns of stimulation is due to the stimulation pattern's ability to disrupt oscillatory and synchronous neural activity. Furthermore the oscillations and synchrony might occur at different frequencies and with different levels of power depending on symptoms, disease progression, medication state, arousal, and intended movement. Methods for generating and applying patient specific, optimal, temporally non-regular patterns of brain stimulation are described herein.

The systems and methodologies disclosed herein include an objective function that maximizes therapeutic benefit (i.e., minimizing the oscillatory power in a particular band of frequencies) and may improve stimulation efficiency by explicitly or implicitly during an optimization protocol to reduce the stimulation frequency, using a model of the subthalamic nucleus (STN) that reproduces the frequency tuning of symptom reduction that has been documented clinically. Furthermore, the stimulation designed by the optimization protocol consists of deterministic, repeating patterns.

According to another aspect of the present teachings, possible solutions to a genetic algorithm (GA) to design novel, repeating non-regular temporal patterns of stimulation that perform well in a validated model of neurological disease pathology.

The only hardware methods available to reduce the artifact for local field potentials (LFPs) is signal filtering (Rossi et al., 2007), but this is not feasible for LFPs during low frequency (<100 Hz) or non-regular patterns of stimulation due to overlap in the frequency domain with the stimulus artifact. According to an aspect of the present teachings, a time-solution may be utilized to mitigate the effect of stimulation artifacts, regardless of the stimulation parameters and pattern.

As described herein, optimized temporally non-regular patterns of stimulation may be used to increase the efficacy and/or efficiency of brain stimulation. The efficacy of these non-regular patterns of stimulation may be due to the ability of the stimulation pattern to disrupt oscillatory and synchronous neural activity. Furthermore, it was observed that the oscillations and synchrony may occur at different frequencies and with different levels of power depending on disease progression, medication state, arousal, and intended movement.

Methods for generating patient-specific, optimal, temporally non-regular patterns of brain stimulation are described. Deep brain stimulation for Parkinson's Disease is used. However, oscillatory and synchronous neural activity is a common feature of many physiological and pathological brain processes, and the present teachings may apply to all situations were brain stimulation may be used to attenuate or potentiate oscillatory or synchronous activity, including, without limitation Essential Tremor, Movement Disorders, Dystonia, Epilepsy, Pain, psychiatric disorders such as Obsessive Compulsive Disorder, Depression, and Tourette's Syndrome.

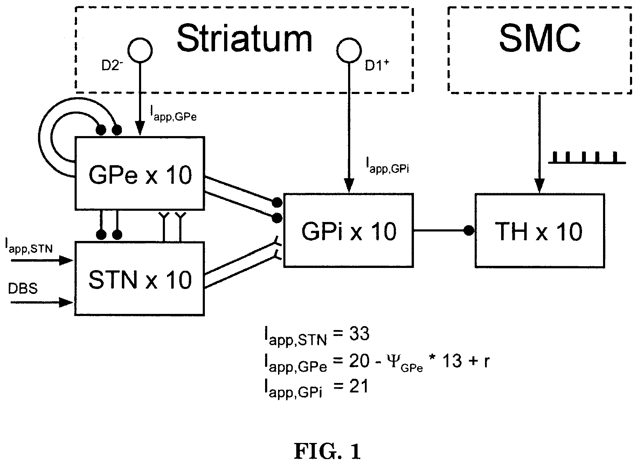

Different types of pathological neural activity are responsible for the different symptoms of neurological disorders treated with brain stimulation. Akinetic motor symptoms in Parkinson's Disease (PD) are associated with oscillatory and synchronous activity in the beta frequency band (10-35 Hz); parkinsonian tremor is associated with low frequency oscillations near the tremor frequency (1-10 Hz); and dyskinesia are associated with synchronized oscillations in the gamma frequency band (around 70 Hz). A computation model of the basal ganglia (Figure 1; a modified version of the So et al., (2012) model), was developed that can reproduce tremor-, akinesia-, and dyskinesia-related oscillations. Therefore, individual symptoms of neurological disorders may be targeted by targeting the underlying pathological neural activity with novel non-regular temporal patterns of brain stimulation, such as those disclosed in U.S. Patent No. 8,447,405.

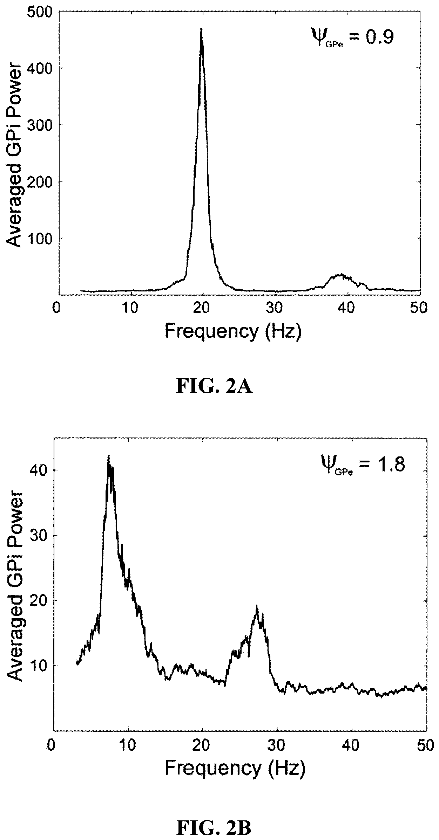

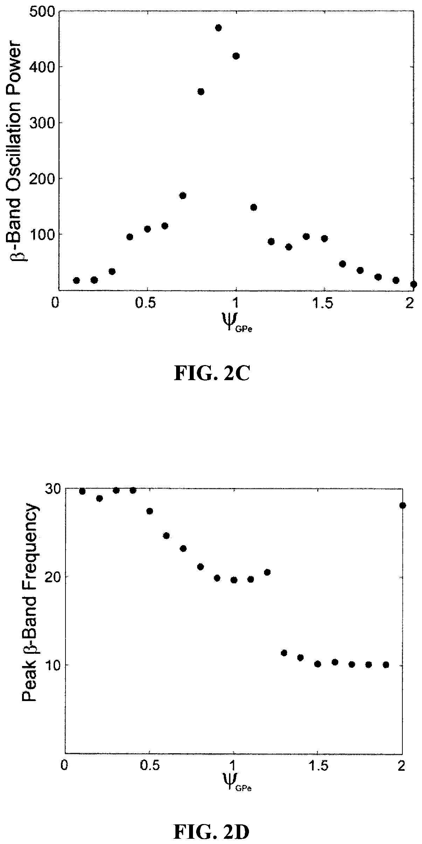

In the model, decreases in dopamine in the striatum may be modeled by decreasing the applied current to external globus pallidus (GPe). As this occurs, the neurons in all nuclei in the model begin to display synchronous oscillations and the frequency and power of these oscillations depend on the amount of current applied to the GPe (Figure 2). For a certain range of current applied to the GPe the model displays synchronous oscillations at beta band frequencies (10-30 Hz). Beta band oscillations and synchronization are present in human patients with PD and are strongly correlated with the presence and resolution by treatment of motor symptoms associated with PD. In other ranges of currents applied to the GPe the neurons begin to show oscillatory/synchronous activity at frequencies associated with tremor in human patients with PD (0-10 Hz). The model can also reproduce dyskinesia-related oscillations in the gamma frequency band if the GPe current is increased to simulate over stimulation with levodopa. The model's neural activity also may show several clinically relevant frequencies of oscillatory/synchronous activity, and thereby enables designing and optimizing patterns of stimulation for the individual symptoms of the neurological disorder. The model predicts that different patients with PD may have different peak-frequencies of oscillations or synchronization depending on their level of dopamine depletion. Therefore, patients may respond differently to specific temporal patterns of brain stimulation that disrupt the oscillatory activity.

### SYMPTOM-SPECIFIC OPTIMIZATION

Symptom-specific design and optimization of non-regular patterns of stimulation may be applied to treat specific patient symptoms as opposed to or in addition to patient specific design and optimization of non-regular patterns of stimulation. Symptom-specific factors to guide optimization, either explicitly in the optimization cost function, or implicitly via modifications of the model characteristics are also described. In an embodiment, non-regular patterns of stimulation may be designed to ameliorate specific symptoms of a neurological disorder (e.g., PD) using a computational model to optimize the patterns of stimulation to suppress oscillations associated with the symptoms. The engineering optimization technique may be the GA, although the present teachings are not limited to this. Any appropriate algorithm may be utilized without departing from the present teachings.

The design and application of symptom-specific brain stimulation parameters were developed using engineering optimization techniques to treat patients categorized by symptom(s). The temporal pattern of stimulation that is delivered to the patient may be selected to ameliorate the patient's symptoms in a desirable manner instead of developing one effective and/or efficient stimulation pattern. According to an aspect of the present teachings, custom patterns of stimulation may be tailored to different combinations of one or more symptoms. A computational model of the relevant brain structures that exhibits the relevant oscillatory/synchronous activity may be used to develop these customized patterns of stimulation. In another aspect of the present teachings, the pattern of stimulation that is delivered may be changed to maintain optimal symptom reduction during changes in medication status, behavioral state, or disease progression.

In an embodiment patients may be categorized according to their symptom(s). The patient may receive a non-regular pattern of stimulation designed specifically to ameliorate that patient's combination of one or more symptoms.

In an embodiment the non-regular patterns of stimulation may be optimized for different symptoms by using engineering optimization techniques in a model that demonstrates activity associated with the symptom(s) of interest.

The engineering optimization techniques may include a GA, and may include a cost function that incentivizes reducing the activity in the model that is associated with a symptom. The cost function could also include increasing types of activity in the computational model thought to offset pathological activity and symptoms. The cost function could also include measures of stimulation energy (e.g., pulse frequency, pulse amplitude, pulse duration, waveform shape, or functions thereof). The cost function could also include physical measurements of symptoms, for example, tremor, rigidity, or bradykinesia. The cost function could also include biomarkers related to disease status or symptom level including functional imaging measures, neurochemical concentrations, or measures of activity from single or multiple neurons (e.g., electroencephalogram).

For example, bradykinesia is a cardinal symptom of PD, and there are different approaches to designing a non-regular temporal pattern of stimulation for this symptom. By way of a non-limiting example, the pattern of stimulation to suppress beta band activity in a computational model may be optimized because beta band neural activity is associated with bradykinesia. Alternatively, a pattern of stimulation to maximize gamma frequency neural activity in the computational model may be optimized. Gamma band activity is associated with prokinetic conditions in PD, and therefore could be useful for patients with bradykinesia. A pattern with two or more optimization criteria, for example a non-regular pattern that is optimized to simultaneously reduce beta band activity and maximize gamma band activity may be utilized.

Since different frequencies of oscillatory/synchronous activity are associated with different symptoms in PD, the stimulation parameters may be selected to treat specific symptoms of the neurological disorder. Tremor in patients with PD is associated with low frequency oscillatory activity in the 1 to 10 Hz range. The model described above displays oscillatory activity in this frequency range, and may be used to optimize stimulation parameters to disrupt low frequency oscillatory neural activity. Oscillatory neural activity with frequencies between 10 to 30 Hz is associated with bradykinesia in patients with PD. The computational model described reproduces oscillatory activity in this frequency range. Therefore, stimulation patterns are designed to effectively disrupt 10 to 30 Hz oscillations in the model's neural activity. In this way, stimulation parameters are optimized to target specific symptoms of the disease.

Patients could receive additional treatment if symptoms change, for example, during disease progression or changes in medication status. Recording the patient's neural activity may guide selection of optimal pattern of stimulation. Recording may include local field potential recorded through the brain stimulation electrode, either during the initial electrode implantation or during implantable pulse generator (IPG) replacement surgery, or at intervals or an a continuous basis by an IPG capable of such recordings. Recordings may also involve single-unit recordings or electroencephalogram (EEG) recordings.

The optimization of temporal patterns of stimulation for specific symptoms may be uncoupled from computational models. Instead optimization techniques may include factors specific to an individual symptom. These factors may include physical measures of the symptoms, either by instrumentation, clinical examination, or clinical rating scales, neural activity related to the symptom(s), energy consumption, imaging results, disease characteristics (including disease progression, stage, and manifesting symptom), and target nucleus.

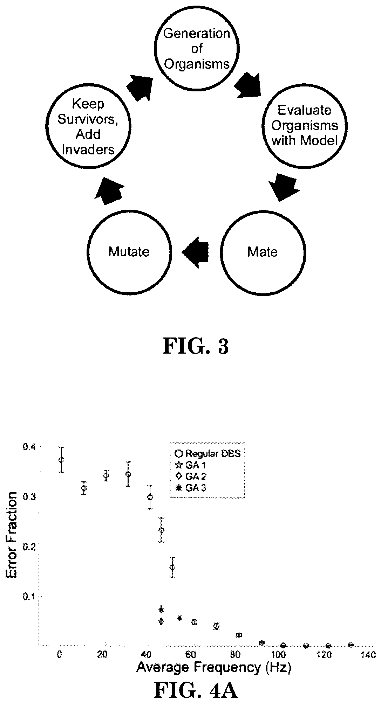

Using deep brain stimulation (DBS) for PD as an example, the non-regular patterns of stimulation may be generated using a computational model of DBS in the subthalamic nucleus (STN). Stimulation patterns may be designed using a model where the current applied to the globus pallidus externus (GPe) is tuned so that the spectral characteristics of the model neuron activity match the neural activity associated with one or more symptoms of PD. In this way, stimulation parameters may be designed to modulate neural activity associated with certain symptoms. The computational model may be combined with an optimization algorithm, for example, the GA, to design these patterns of stimulation. The progression of a GA is illustrated in Figure 3.

There are several important characteristics of the GA that should be highlighted. First, there is the unique deterministic encoding of patterns of stimulation in the current GA such that the GA is directly optimizing the repeating patterns of stimulation, and not optimizing a stochastic process that could create effective non-regular stimulation. Second, there is the use of a cost function in a validated model of neurological disorder pathology. The current GA implicitly forces patterns of stimulation toward lower average frequencies by defining the cost as the percent change in the patterns performance compared to a frequency matched regular DBS control.

Although an embodiment of the present teachings used an evolutionary algorithm, namely a GA, the present teachings are not limited to GAs. All model-based optimization techniques including, but limited to, other evolutionary algorithms, swarm intelligence algorithms, and other optimization techniques may be used.

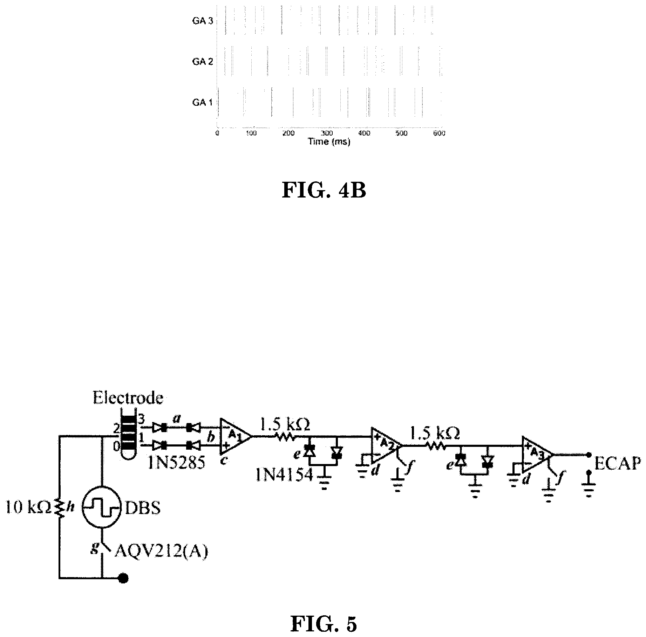

The scope of the present teachings shall not be limited to this particular model of the PD state or to any set of models of neurological disorders. Present or future models of neurological disorders that are treated with brain stimulation, currently or in the future, are candidates for use with the methods described in these teachings. Furthermore, these teachings are not limited to a particular pattern or set of patterns generated by the methods described-here. A few exemplary patterns of stimulation designed by the GA are shown in Figure 4.

In simulation experiments, optimal temporal patterns of stimulation were designed for a subset of currents representative of different symptoms that were applied to the GPe. This resulted in decreased oscillatory activity in the selected brain region or nucleus as described below and depicted in, for example, Figure 2 and 4.

The present teachings described are implemented in an implantable pulse generator capable of producing specific patterns of the non-regular stimulation. The device may also record local field potentials or other neural activity so that the device may deliver the temporal pattern of stimulation that is most effective for the type of neural activity recorded. The device may be configured to be programmed to deliver such applicable temporal pattern of stimulation.

### OBTAINING NEURAL RECORDINGS

An implantable pulse generator may generate and deliver non-regular patterns of stimulation while simultaneously recording neural activity. The implantable pulse generator may use an amplifier-blanking paradigm that briefly grounds the inputs during a short period encompassing the stimulation pulse. This prevents violating the input specifications of the amplifiers (railing the amplifiers), and the short gaps in the data may be overcome with real-time or post-processing analysis methods described below.

The recorded neural activity may be used to monitor performance of the stimulation pattern; control when the stimulation pattern is applied; trigger switches between pre-programmed patterns of stimulation; control interleaving between different stimulation patterns; and/or allow for *in vivo* optimization of the temporal pattern of stimulation.

The device and methods described herein are not limited to application of any particular type of stimulation. However, the device and methods may be especially useful during non-regular temporal patterns of stimulation, because there is strong impetus for using non-regular patterns of stimulation for the treatment of patients with neurological disorders. Mechanisms exist at the cellular and systems level to explain the effectiveness of specific temporal patterns of stimulation.

At a cellular level, the use of non-regular stimulation of the nervous system relies on the sensitivity of neurons to the specific timing of stimulation pulses. In other words, if the specific timing of the stimulation is important to individual neurons or even a population of neurons, it may be advantageous for DBS systems to use non-regular temporal patterns of stimulation to exploit this sensitivity. In the branch of neuroscience concerned with the neural code (i.e., how neurons communicate information with one another), the importance or the timing of inputs to a neuron as it relates to information transfer in the system is a common idea that is termed temporal (or spatiotemporal) coding.

At a systems level a non-regular stimulation pattern could be more effective than regular stimulation at disrupting or reversing pathological features or a neurological disorder such as PD. For example, a non-regular pattern of stimulation may be able to disrupt pathological synchronization and oscillations that are common in systems affected by PD.

Exploiting the neural coding by taking advantage of the brain's sensitivity, at any level, to the temporal structure of stimulation makes the technology described here different from other stimulation protocols developed to treat neurological disorders.

Because the primary goal of brain stimulation is to modulate neural activity in the brain, recording neural activity during brain stimulation enables evaluation of the effects of stimulation on neural activity. Further, it can guide application, selection, and optimization of stimulation parameters, such as the pattern of stimulation, either intermittently or continually.

The primary challenge to making these neural recordings is separating the desired neural signal from the deleterious stimulation artifact. Local field potentials (LFPs) and electrically evoked compound action potentials (ECAPs) may be the neural activity of interest, and device implementation to record these two signals is discussed. separately below.

### Electrically Evoked Compound Action Potentials

Recording ECAPs is challenging due to the large stimulus artifact that can cause amplifier saturation and mask the ECAP signal (Rossi et al., 2007; McGill et al., 1982). Described herein is DBS-ECAP instrumentation that may limit the stimulus artifact and enable high fidelity recording of short latency, small amplitude ECAP signals. Figure 5 is a diagram of the DBS-ECAP instrumentation used for stimulus artifact reduction and ECAP recording during DBS. As shown in Figure 5, anti-series, current-limiting diodes (1N5285) were connected to the DBS lead prior to the amplification stages (a); differential recordings were made from DBS contacts 0 and 2 (b), and served as inputs to the preamplifier (A₁) (c). Two additional series amplifier stages (A₂ and A₃) further increased the gain and filtered the signal with 10 Hz to 10kHz pass-band (d). Anti-parallel diodes (1 N4154) were placed at the inputs A2 and A3 (e). During each stimulus pulse, an opto-isolated CMOS multiplexer (74HC4053) internally grounded the signal path in amplifiers A2 and A3 (e). A PhotoMOS relay (AQV212(A)) disconnected the stimulating electrodes in between DBS pulses (g). The parallel resistances enabled any accumulated charge on the stimulating electrodes to discharge between DBS pulses, and enabled near-critical damping of the signal recovery from artifact to baseline (h). Monopolar stimulation was delivered between DBS contact 1 and a distant return electrode.

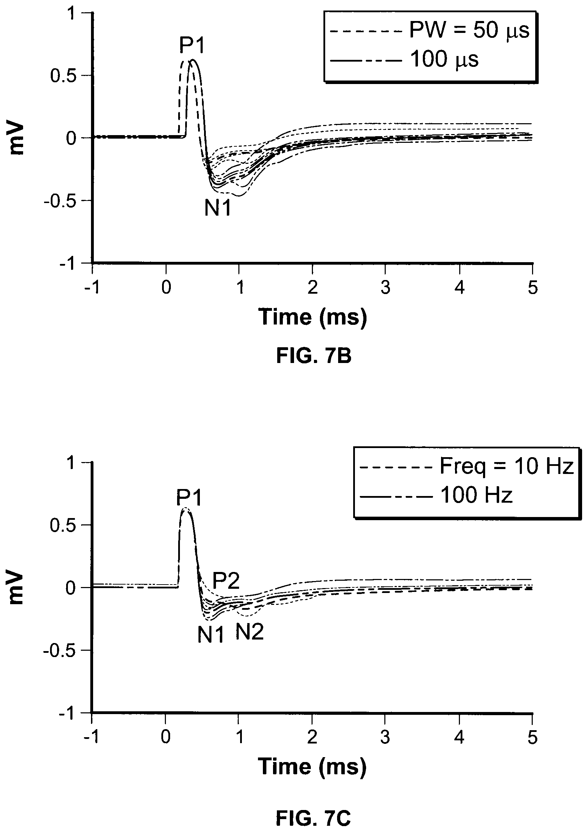

Figures 7A-7C depict *in vivo* ECAP responses measured across stimulation parameters and recorded from a mini DBS electrode implanted in the thalamus of a cat using the DBS-ECAP instrumentation. For each of Figures 7A- 7C, individual ECAP responses and the average ECAP waveform are shown. Figure 7A shows ECAP responses for DBS amplitudes between 1 and 3 V. Figure 7B shows pulse widths of 50 and 100 µs/phase. The magnitude and duration of the early positive (P1) and negative (N1) waves generally increased with DBS amplitude and pulse width. Figure 7C shows ECAP responses for DBS frequencies of 10 and 100 Hz. Secondary positive (P2) and negative (N2) waves were generated at 10 Hz but decayed during the 100 Hz DBS train and were not present in the average signal.

Recordings may be made from two non-stimulating contacts on the four contact DBS electrode, which may eliminate the need for additional recording electrodes and help ensure that the recording contacts are near the neurons activated by stimulation. The recording contacts serve as inputs to the DBS-ECAP instrumentation, which uses stages of series amplification and several circuit components to reduce the stimulus artifact (Kent and Grill, 2012). Amplifier blanking and a stimulus relay circuit may be used to suppress the stimulus artifact and its duration.

In an embodiment, the first stage may utilize a battery-powered preamplifier (Al, SRS60, Stanford Research Systems), which may measure the differential signal from the recording contacts to reduce common mode noise, and provide gain and high input impedance. Two additional amplifier stages (A2 and A3, SR560) may be placed in series to increase gain further and to filter the signal with a 10 Hz to 10 kHz pass-band. Anti-parallel diode clamps (1N4154, Fairchild Semiconductor) may be placed at the inputs of A2 and A3 to ground the line if the input voltage exceeds approximately ±0.7 V, thereby selectively clipping the stimulus artifact and enabling increased gain without saturation. To achieve further increases in gain, the signal paths in amplifiers A2 and A3 may be internally grounded through an opto-isolated CMOS multiplexer (74HC4053), blanking the output for the duration of each stimulus pulse and a subsequent 1 00µs. The rapid turn-off time of this CMOS switch (10 µs) may ensure that short latency ECAP responses may still be recorded. In addition, a low-resistance, rapid-response Photo MOS relay (AQV212(A), Panasonic) may disconnect the stimulating electrodes between DBS pulses. This may limit capacitive discharge from the electrode-tissue interface through the stimulator after each pulse, and thereby may reduce the duration of the stimulus artifact (McGill et al, 1982). A 10 kS2 parallel resistor may be placed across the stimulating electrodes to allow accumulated charge on the stimulating contacts to discharge between pulses. Further, this resistor may enable near critical damping of the signal recovery from artifact to baseline. The digital pulse controlling the closing of the stimulator relay may be turned off approximately 40 ps before the end of the DBS pulse to account for the intrinsic delay of the relay. The digital pulses controlling the amplifier blanking and closing of the stimulator relay may be turned on approximately 2 ms before each DBS pulse to account for turn on delays, and to discharge any charge remaining on the stimulating electrodes.

### Local Field Potentials

There may be no guarantee of separation between the LFPs (typically interested in frequencies less than 100 Hz) and the stimulation artifact in the frequency domain during non-regular patterns of stimulation, and even during low frequency (<100 Hz) regular stimulation. Therefore, frequency-domain solutions, such as filtering, may not be effective for mitigating the effects of stimulation artifacts. If the spectral content of the LFPs is of interest, then the non-regular stimulation artifact may also introduce power across the range of frequencies contained within the stimulation train. Therefore, a time-domain solution may be incorporated into an implantable pulse generator device. Grounding or blanking the signal recorded from the unused electrode contacts during the stimulation pulse and possibly the time immediately preceding and following the pulse may be utilized.

In an embodiment, an amplifier blanking paradigm such as the one described above for the ECAP instrumentation may be used. The blanking of the signal may introduce "gaps" into the LFP signals, but these gaps can be overcome with data processing techniques discussed below. It is important that low pass filtering be avoided in any stage before the blanking of the stimulation artifact, because any low pass filtering may prolong the duration of the artifact signal and require an increased blanking epoch after the stimulation pulse is delivered.

Some of the device capabilities include, but are not limited to, delivering non-regular temporal patterns of stimulation; delivering regular temporal patterns of stimulation; utilizing a range of stimulation parameters, including pulse widths, pulse waveforms, average stimulation frequency, and applied voltages or currents; recording neural activity through electrode contacts during stimulation-off epoch; including electrically evoked compound action potentials and local field potentials; recording neural activity during stimulation-on epoch through unused electrode contacts, including during non-regular temporal patterns of stimulation; differential and single-ended recordings; appropriate signal amplification; signal conditioning and filtering; using diodes to clip high amplitude input signals; intermittently blanking input signal; using a programmable method to shorten the stimulus artifact during recording of stimulation evoked neural activity such as ECAPS; on-board data processing of recorded neural activity; real-time or post hoc optimization or learning of stimulation parameters, including temporal pattern of stimulation; telemetry with devices outside of patient's body; and pattern optimization during telemetry sessions with device running optimization/learning algorithms. In an embodiment the device may be an implantable, hermetically sealed-device. In an embodiment, the device may include an on-board power source (e.g., a rechargeable or non-rechargeable battery). In an embodiment the device may use as short of a pulse as possible (e.g., symmetric biphasic pulses), which may reduce the duration of time the input is blanked. In an embodiment the input signal may be blanked during the stimulation pulse and possibly for a short period of time before and/or after the stimulation pulse. In an embodiment, the device may include a programmable blanking configuration that could be used to record different types of neural signals. In an embodiment, the device may include a stimulation relay paradigm as depicted in Figure 5.

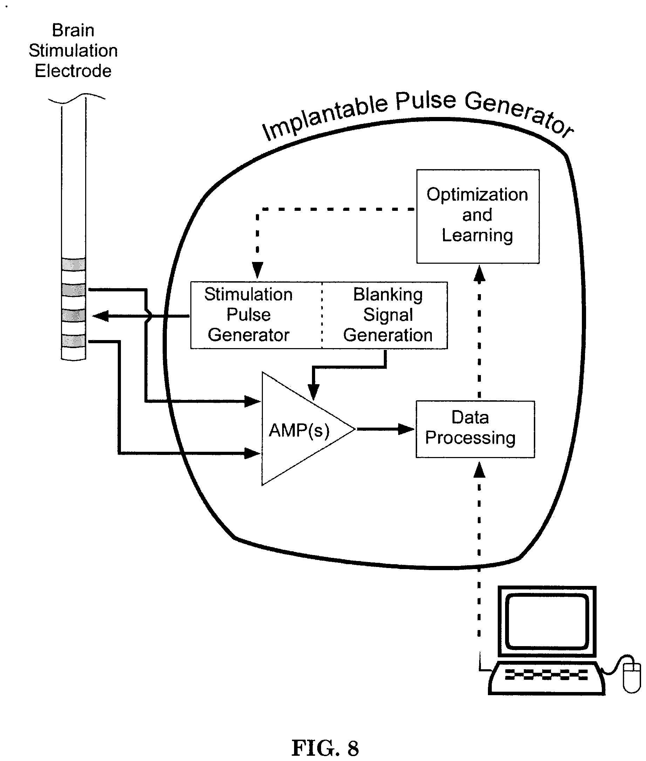

Figure 8 depicts an embodiment of the present teachings in which the device may include stimulation pulse generation components coupled to a blanking signal generation module. The blanking signal may be sent to the amplifier/filtering module (AMP(s)) to perform the signal blanking described above. A data processor may perform required data processing (described below). This module may have the ability to communicate wirelessly with devices outside the patient's body. The optimization and learning module may use the processed data to control stimulation parameters.

### Data Processing Methods

Several different data processing techniques can be employed to overcome the gaps in the data and extract signal characteristics of interest. For example, if evoked neural activity is of interest, the gaps are not troublesome and data could be averaged over several stimulation pulses to achieve a measure of the evoked activity. If continuous neural activity is of interest, then the gaps are troublesome, but can be overcome. There are two main approaches: 1) fill in the gaps with modeled data, and 2) work around the gaps while estimating characteristics of interest.

Several methods for filling in the gaps with proxy data are described herein without limitation. One of the simplest techniques is to use linear interpolation within the gaps to join the data points before and after the gaps. This method may introduce bias depending on the signal characteristics of interest. For example, signal spectral characteristics are the preferred signal characteristic and linear interpolation will introduce bias into the spectrum estimate. Another data processing option is to fill in the gaps with data generated by a model trained on data before and/or after the gap. For example, data generated by the autoregressive (AR) model will have the same characteristics (spectral and otherwise) as the data the model was trained on, and may produce a good proxy for the real data (Walter et al., 2012). Because the data generated by the AR model is not guaranteed to meet the data at the end of the gap, linear interpolation may be used in combination with AR modeling to mitigate the chance of jumps in the reconstructed signal. Any method for calculating the AR model (e.g., least squares, Burg's algorithm, etc.) may be used, and that other types of models may be used to fill in the gaps with data. Further, data segments may simply be appended together to eliminate the gaps. •

The other data processing approach may be to work around the gaps and directly estimate the statistics or signal characteristics of interest. If the spectral content of the recorded signal is of interest, one may bypass reconstructing the data in the gaps entirely and instead train an AR model on the data around the gaps and calculate the power spectrum calculated directly from the model (Walter et al., 2012). Several other methods for data analysis exist that may enable working around the gaps in the recorded signal while still extracting the information of interest without introducing bias.

### Application of Recorded Neural Signals

The recorded neural activity can be used purely for monitoring purposes and indicate the efficacy of the stimulation. The recorded activity or summary statistics from the recordings can be downloaded from the device by a healthcare provider, company representative, device programmer, certified research scientists, or any appropriate person. The recorded neural activity can be used to guide intermittent or continuous modulation of stimulation parameters. Non-regular temporal patterns of stimulation can be demand-controlled, and stimulation may remain off when not needed (e.g., when the patient is asleep). The recorded neural activity can also be used as a trigger or indicator for switching between pre-programmed temporal patterns of stimulation. These different patterns may have different levels of energy efficiency, efficacy, or be targeted for different situations (e.g., On/Off medications) or be targeted for different symptoms (e.g., tremor or bradykinesia).

The ECAP signals can be coupled with a computational model to determine which neural elements generate the ECAP signatures and thereby mediate the beneficial treatment effects of DBS. Stimulation parameters can be modified to improve the efficacy of treatment by enabling targeted stimulation of the neural elements that produce the desired response.

The recorded neural activity can be used to guide *in vivo* optimization or learning algorithm based modulation of the temporal pattern of stimulation. Non-regular patterns of stimulation can be built one interpulse interval at a time based on the recorded neural activity. Alternatively, engineering optimization algorithms such as a GA can be used to design non-regular patterns of stimulation. Also, a control system may be used to guide the temporal pattern or stimulation. Lastly, machine learning algorithms can be used to learn the pattern of stimulation that meets the stimulation objectives (based on the recorded neural activity) most effectively. This can take place in real time.

Any *in vivo* optimization/learning or temporal patterns of stimulation includes safety features to prevent undesired stimulation parameters or uncomfortable side effects. There is a defined period of time when the optimization runs, which could be after the initial electrode and pulse generator implantation or periodically thereafter.

Non-regular temporal patterns of stimulation can be updated and optimized intermittently to meet stimulation objectives (e.g., suppress recorded pathological patterns of neural activity while minimizing energy usage). Further, the continuous recording or neural activity allows real-time optimization of the non-regular pattern or stimulation via an automated optimization algorithm incorporated into the implantable pulse generator.

The application of ECAPs to DBS parameter optimization and closed-loop systems disclosed herein. ECAPs may indicate how neurons respond during stimulation, and so may better reveal the mechanisms of action of DBS and serve as a reliable feedback signal for brain stimulation.

Other available techniques used to remove the stimulus artifact may be inadequate for ECAP or LFP recording during brain stimulation, especially for non-regular pattern brain stimulation.

### Patient-Specific Optimization

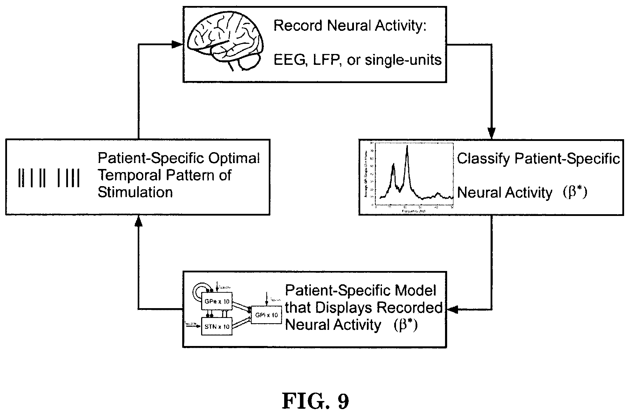

The design and implementation of optimal temporally non-regular patterns of brain stimulation to disrupt pathological oscillatory/synchronous activity is described herein. Patient-specific recordings of neural activity can be used to guide patient-specific optimization of brain stimulation parameters, including optimal temporal patterns of stimulation. Recordings taken from the patient characterize the oscillatory/synchronous neural activity. A model, such as the one described above, may be tuned to reproduce the observed oscillatory/synchronous neural activity. This patient-specific model may be used to develop or optimize the parameters of stimulation (Figure 9). Stimulation parameters, including the temporal pattern, may be chosen or optimized to disrupt or- promote oscillatory or synchronous activity based on the neural activity that a specific patient exhibits. While the present teachings may be applicable to myriad neurological disorders, PD may involve disrupting beta band activity; and because each patient's neural activity may have slightly different spectra, patient-specific stimulation parameters may more effectively disrupt pathological activity than one-size-fits-all parameters.

Because different frequencies of oscillatory/synchronous activity are associated with different symptoms in PD, the stimulation parameters may be selected to treat specific symptoms of a neurological disorder. Tremor in patients with PD is associated with low frequency oscillatory activity in the 1-10 Hz range.

The model described herein may be capable of exhibiting oscillatory activity in this frequency range, and may be used to optimize stimulation parameters to disrupt low frequency oscillatory neural activity. Oscillatory neural activity with frequencies between 10 and 30 Hz may be associated with bradykinesia in patients with PD. The computational model described herein may also be capable of reproducing oscillatory activity in this frequency range. Therefore, stimulation patterns may be designed to effectively disrupt 10 to 30 Hz oscillations in the model's neural activity. In this way, stimulation parameters can be optimized to target specific symptoms of the disease.

Patients may receive "tune-ups" if the frequency or characteristics of their oscillatory/synchronous activity changes, for example, during disease progression or changes in medication status. Recording the patient's neural activity may involve local field potential recordings through the brain stimulation electrode, either during the initial electrode implantation or during IPG replacement surgery, or at intervals or on a continuous basis by an IPG capable of such recordings. Recordings may also involve single-unit recordings or EEG recordings.

The present teachings, therefore, describe the selection of specific stimulation parameters and temporally non-regular patterns of stimulation according to the neural activity recorded from a particular patient using a patient-specific computational model that reproduces the observed neural activity.

In the present example of DBS for PD, the non-regular patterns of stimulation may be generated using a computational model of DBS in the STN. Stimulation patterns may be designed using a model where the current applied to the GPe is tuned so that the spectral characteristics of the model neurons' activity generally match the patient's recorded neural activity. In this way, different stimulation parameters may be designed to modulate neural activity with specific spectral characteristics. The computational model may be combined with an optimization algorithm, for example, the GA, to design these patterns of stimulation. The progression of a GA is illustrated in Figure 3. Resulting patterns of non-regular stimulation can be tested using an intraoperative experiment.

There are several important characteristics of the GA that should be highlighted. First, there is the unique deterministic encoding of patterns of stimulation in the GA such that the GA is directly optimizing the repeating patterns of stimulation, and not optimizing a stochastic process that may create effective non-regular stimulation. Second, there is the use of a cost function in a validated model of neurological disorder pathology. The current GA implicitly forces patterns of stimulation toward lower average frequencies by defining the cost as the percent change in the patterns performance compared to a frequency matched regular DBS control. It will be appreciated that optimization algorithm is not limited to GAs. All model-based optimization techniques including, but limited to, other evolutionary algorithms, swarm intelligence algorithms, and other optimization techniques may be used.

It will also be appreciated that the present teachings shall not be limited to any particular model of the PD state or to any set of models of neurological disorders. Present or future models of neurological disorders that are treated with brain stimulation, currently or in the future, are candidates for use with the described system and method.

Furthermore, the present teachings are not limited to a particular pattern or set of patterns generated by the methods described here. Examples of patterns of stimulation designed by the GA are shown in Figure 10. It will be appreciated that any pattern or set of patterns may be used for the system and method described herein.

In an embodiment an IPG capable of producing specific patterns of the non-regular stimulation may used. The device may record local field potentials or other neural activity so that the device may deliver the temporal pattern of stimulation that is most effective for the type of neural activity recorded.

Patient specific brain stimulation parameters may be designed based on recorded neural activity. The temporal pattern of stimulation that is delivered to the patient may be selected to modulate the patient's neural activity in a desirable manner. Instead of developing a handful of effective and/or efficient stimulation patterns, custom patterns of stimulation may be tailored to each individual patient. A computational model of the relevant brain structures that exhibits the relevant oscillatory/synchronous activity may be used to develop these customized patterns of stimulation. The pattern of stimulation delivered may be changed to maintain optimal symptom reduction during changes in medication status, behavioral state, or disease progression. For example, recordings of patient specific brain oscillatory activity during wakeful rest, activity, and sleep, can be used to design specific optimal temporal patterns of stimulation for of each these states.

There is evidence that oscillatory and synchronous neural activity may be the cause of many neurological disorders and may be important for the proper functioning of brain structures. There is also evidence that the spectral characteristics of neural activity differ across patients or change over time. Therefore, the invention described herein is useful to patients with neurological disorders because it may provide customized stimulation parameters based on their neural activity. The customized stimulation may be more effective and/or efficient than regular high frequency stimulation and/or some other stimulation patterns. Furthermore, the present teachings may allow the temporal pattern of stimulation to be adjusted as changes in neural activity occur, perhaps because of disease progression.

### LITERATURE CITATIONS

Alvarez I, et al., "Generalized alternating stimulation: a novel method to reduce stimulus artifact in electrically evoked compound action potentials. J Neurosci Methods 165:95-103 (2007).
Bahmer A, et al., "Recording and analysis of electrically evoked compound action potentials (ECAPs) with MED-EL cochlear implants and different artifact reduction strategies in Matlab." J. Neurosci Methods 191:66-74 (2010).
Birdno M J "Analyzing the mechanisms of thalamic deep brain stimulation: computational and clinical studies". Ph.D. Dissertation. Department of Biomedical Engineering, Duke University, Durham, N.C., USA, August 2009.
Brocker DT, Swan BD, et al., "Improved efficacy of temporally non-regular deep brain stimulation in Parkinson's disease." Experimental Neurology, 239:60-67. (2013)
Brown CJ, Abbas PJ "Electrically evoked whole-nerve action potentials: parametric data from the cat." JAcoust Soc Am 88:2205-10 (1990),
Dorval AD, Kuncel AM, et al. "Deep Brain Stimulation Alleviates Parkinsonian Bradykinesia by Regularizing Pallidal Activity." I Neurophysiol 104(2): 911 .921, (2010).
Feng X J, Shea-Brown E, Greenwald B, Kosut R, Rabitz H Optimal deep brain stimulation of the subthalamic nucleus-a computational study. J Comput Neurosci. 23(3):265-282 (2007).
Grill WM, Dorval AD. "Non-regular electrical stimulation patterns for treating neurological disorders", US Patent Application No. 2010-0152807, flied 5 October 2009*.*
Hashimoto T, Elder CM, Vitek JL "A template subtraction method for stimulus artifact removal in high-frequency deep brain stimulation." J. Neurosci Methods 113:181-6, (2002*).*
Jeon EK, Brown CJ, Etler CP, O'Brien S, Chiou LK. Abbas PJ " Comparison of electrically evoked compound action potential thresholds and loudness estimates for the stimuli used to program the Advanced Bionics cochlear implant." Am Acad Audiol21:16-27. (2010*).*
Kent AR, Grill WM "Recording evoked potentials during deep brain stimulation: development and validation of instrumentation to suppress the stimulus artifact." Journal of Neural Engineering 9(3):036004 (2012*).*
McGill KC, Cummins KL, Dorfman LJ, Berlizot BB, Leutkemeyer K, Nishimura DG, Widrow B. "On the nature and elimination of stimulus artifact In nerve signals evoked and recorded using surface electrodes" IEEE Trans Biomed Eng 29:129-37 (1982).
Miller CA, Abbas PJ, Brown CJ (2000). "An improved method of reducing stimulus artifact in the electrically evoked whole-nerve potential" Ear Hear 21:280-90 (2000*)*
Miller CA, Abbas PJ, Robinson BK, Rubinstein JT, Matsuoka AJ "Electrically evoked single-fiber action potentials from cat: responses to monopolar, monophasic stimulation." Hear Res 130:197-218. (1999*).*
Miller CA, Brown CJ, Abbas PJ and Chi SL "The clinical application of potentials evoked from the peripheral auditory system." Hear Res 242:184-97 (2008).
Quinkert AW, Pfaff DW "Temporal patterns of deep brain stimulation generated with a true random number generator and the logistic equation: effects on CNS arousal in mice." Behav Brain Res 229(2):349-58 (Apr. 15, 2012)
Rossi L, Foffani G, Marceglia S, Bracchi F, Barbieri S, Priori A "An electronic device for artifact suppression in human local field potential recordings during deep brain stimulation." JNeuralEng 4:96-106. (2007).
So RQ, Kent AR, Grill WM. "Relative contributions of local cell and passing fiber activation and silencing to changes in thalamic fidelity during deep brain stimulation and lesioning: a computational modeling study:" J Computational Neurosci 1-21. (2011)
Stypulkowsi PH, van den Honert C "Physiological properties of the electrically stimulated auditory nerve. I. Compound action potential recordings." Hear Res 14:205-23 (1984)
Taylor Tavares AL, Jefferis GSXE, et al. "Quantitative measurements of alternating finger tapping In Parkinson's disease correlate with UPDRS motor disability and reveal the improvement in fine motor control from medication and deep brain stimulation." Movement Disorders 20(10): 1286 .1298. (2005).
Wagenaar DA,. Potter SM. "Real-time multi-channel stimulus artifact suppression by local curve fitting." J Neurosci Methods 120:113-20. (2002)
Walter A, et al., "Coupling BCI and cortical stimulation for brain-state-dependent stimulation: methods for spectral estimation in the presence of stimulation alter-effects." Frontiers in neural circuits 6: DOJ=10.3389/fncir.2012.00087 (2012).

## Claims

1. A method for generating a brain stimulation pattern for treating a patient with a neurological disorder, wherein the neurological disease is Parkinson's Disease (PD), the method comprising:
identifying at least one symptom of the neurological disorder, wherein the at least one symptom of the neurological disorder is selected from the group consisting of akinetic motor symptoms and parkinsonian tremor; and
optimizing a computational model of the brain stimulation pattern to suppress oscillations in a model associated with the at least one symptom of the neurological disorder,
wherein the computational model of the brain stimulation pattern displays synchronous oscillations based upon current applied to an external globus pallidus (GPe) provided in the computational model,
wherein
(i) in case the at least one symptom of the neurological disorder is an akinetic motor symptom, the computational model of the brain stimulation pattern displays synchronous oscillations in the beta frequency band of 10 to 30 Hz; and
(ii) in case the at least one symptom of the neurological disorder is parkinsonian tremor, the computational model of the brain stimulation pattern displays synchronous oscillations in the frequency band of 1 to 10 Hz.

2. The method of Claim 1, wherein the brain stimulation pattern is a non-regular, non-random temporal brain stimulation pattern.

## Patentansprüche

1. Verfahren zum Erzeugen eines Hirnstimulationsmusters zur Behandlung eines Patienten mit einer neurologischen Störung, wobei es sich bei der neurologischen Erkrankung um die Parkinson-Krankheit (PD) handelt, wobei das Verfahren umfasst:
Identifizieren mindestens eines Symptoms der neurologischen Störung, wobei das mindestens eine Symptom der neurologischen Störung aus der Gruppe, bestehend aus akinetischen motorischen Symptomen und Parkinson-Tremor, ausgewählt ist; und
Optimieren eines Rechenmodells des Hirnstimulationsmusters, um Schwingungen in einem Modell zu unterdrücken, die mit dem mindestens einen Symptom der neurologischen Störung verbunden sind,
wobei das Rechenmodell des Hirnstimulationsmusters synchrone Schwingungen auf der Grundlage von Strom anzeigt, der an einen im Rechenmodell bereitgestellten externen Globus Pallidus (GPe) angelegt wird,
wobei
(i) falls das mindestens eine Symptom der neurologischen Störung ein akinetisches motorisches Symptom ist, das Rechenmodell des Hirnstimulationsmusters synchrone Schwingungen im Beta-Frequenzband von 10 bis 30 Hz zeigt; und
(ii) falls das mindestens eine Symptom der neurologischen Störung Parkinson-Tremor ist, das Rechenmodell des Hirnstimulationsmusters synchrone Schwingungen im Frequenzband von 1 bis 10 Hz zeigt.

2. Verfahren nach Anspruch 1, wobei das Gehirnstimulationsmuster ein nicht regelmäßiges, nicht zufälliges zeitliches Gehirnstimulationsmuster ist.

## Revendications

1. Procédé de génération d'un schéma de stimulation cérébrale pour traiter un patient avec un trouble neurologique, dans lequel la maladie neurologique est la maladie de Parkinson (PD), le procédé comprenant :
identifier au moins un symptôme du trouble neurologique, dans lequel l'au moins un symptôme du trouble neurologique est sélectionné parmi le groupe constitué de symptômes moteurs akinétiques et d'un tremblement parkinsonien ; et
optimiser un modèle informatique du schéma de stimulation cérébrale pour supprimer des oscillations dans un modèle associé à l'au moins un symptôme du trouble neurologique,
dans lequel le modèle informatique du schéma de stimulation cérébrale affiche des oscillations synchrones basées sur du courant appliqué à un globus pallidus externe (GPe) prévu dans le modèle informatique,
dans lequel
(i) au cas où l'au moins un symptôme du trouble neurologique est un symptôme moteur akinétique, le modèle informatique du schéma de stimulation cérébrale affiche des oscillations synchrones dans la bande de fréquences bêta de 10 à 30 Hz ; et
(ii) au cas où l'au moins un symptôme du trouble neurologique est un tremblement parkinsonien, le modèle informatique du schéma de stimulation cérébrale affiche des oscillations synchrones dans la bande de fréquences de 1 à 10 Hz.

2. Procédé selon la revendication 1, dans lequel le schéma de stimulation cérébrale est un schéma de stimulation cérébrale temporale non aléatoire, non régulière.
